# FASCICULE DE BREVET EUROPEEN

(11) **EP 0 754 033 B1**
(45) Date de publication et mention de la délivrance du brevet: **03.11.1999**
(21) Numéro de dépôt: 95916694.3
(22) Date de dépôt: 31.03.1995
(51) Int. Cl.: A61K 9/24, A61K 31/40

(54) **COMPOSITION GALENIQUE MUCOADHESIVE HETEROFONCTIONNELLE**
HETEROFUNKTIONELLE MUCOADHÄSIVE GALENISCHE ZUSAMMENSETZUNG
HETEROFUNCTIONAL, MUCOADHESIVE DOSAGE FORM

(30) Priorité: 01.04.1994 FR 9403895
(43) Date de publication de la demande: 22.01.1997
(73) Titulaire: Biotec Centre S.A., 45071 Orléans Cédex 2 (FR)
(72) Inventeur: BROMET, Norbert Emile, F-45100 Orléans-la-Source (FR)
(74) Mandataire: Desaix, Anne
(86) Numéro de dépôt international: FR9500417
(87) Numéro de publication internationale: WO9526713

(56) Documents cités:
- EP-A- 0 020 777
- EP-A- 0 159 604
- EP-A- 0 518 468
- WO-A-87/04342
- WO-A-91/06290
- US-A- 4 250 163

## Description

La présente invention est relative à une formulation galénique permettant d'administrer des produits tels la mélatonine ou ses dérivés en associant une dose de charge permettant une action rapide et dans le cas de la mélatonine (ou de ses dérivés) un signal chronobiologique clair et d'une ou plusieurs doses libérées progressivement, permettant d'assurer une mise à disposition de produits dans l'organisme rapidement, et de façon prolongée.

De façon générale, la présente invention est relative à toute formulation mucoadhésive associant au moins deux formulations à libérations contrôlées de vitesses différentes, depuis une forme rapide jusqu'à une forme à libération prolongée.

Le produit est particulièrement avantageux lorsque la formulation est appliquée à une administration conjointe par voie transmucosale ou sublinguale et par voie orale ou la composition galénique associe une forme orale rapide à une forme orale à libération contrôlée et une forme transmucosale au sein d'un seul comprimé buccal mucoadhésif.

La mélatonine (N-acetyl-5-méthoxytryptamine) est une hormone endogène synthétisée par l'épiphyse (ou glande pinéale) et impliquée entre autre dans la régulation du rythme circadien, dans l'inhibition du développement gonadique et dans la régulation de l'ovulation.

Elle présente la particularité d'être libérée pendant la nuit uniquement chez les mammifères, et en particulier chez l'homme.

Dans l'organisme, cette molécule est catabolisée dans le foie et les reins pour former entre autre la 6-hydroxy-mélatonine.

Une caractéristique importante de la mélatonine est son rythme circadien de sécrétion très marqué ; en effet, les taux plasmatiques de cette hormone sont faibles pendant le jour (inférieurs à 10 pg/ml) et sont élevés pendant la nuit (de l'ordre de 70 pg/ml) chez l'homme (1, 2).

La relation entre le cycle de la mélatonine, c'est-à-dire ses variations de concentrations dans le plasma et le cycle du sommeil, n'est pas claire, mais la mélatonine peut aider à organiser ou réorganiser les cycles circadiens.

Dans la présente demande, la mélatonine et ses dérivés seront utilisés et décrits comme principes actifs administrés dans la composition galénique mucoadhésive hétérofonctionnelle.

Elle représente un exemple particulièrement significatif du fait des caractéristiques mêmes de l'action de ce composé qui doit à la fois atteindre rapidement la circulation systémique et présenter concomitamment une libération progressive permettant ainsi une mise à disposition immédiate, puis prolongée du produit.

Tout type de composé comme les dérivés de la mélatonine substitués en 2 ou en 6 par des halogènes, ou encore tout autre dérivé tel que décrit dans les demandes de brevets WO88/07367 ou WO89/04659 ou d'autres substances à effet pharmacologique nécessitant à la fois une libération rapide allant d'une libération de type flash jusqu'à une libération de type lent et contrôlé peuvent être avantageusement introduites dans la formulation galénique de l'invention.

La mélatonine et ses dérivés, compte tenu de leur caractéristique d'être un facteur de régulation du système circadien, est utilisable par exemple lors d'un voyage transméridien pour traiter le phénomène de "jet lag" (3). Pendant cette période, la plupart des personnes souffrent entre autres symptômes de troubles de l'humeur, de perturbation du sommeil, de modification de la vigilance au cours de la journée ; différentes études montrent effectivement une amélioration des symptômes de décalages horaires après un traitement à base de mélatonine ; plus récemment, il a été montré que des sujets qui subissaient des décalages horaires de 6 à 12 heures montraient une resynchronisation plus rapide des différents rythmes circadiens (6-sulphatoxymélatonine, cortisol, température, électrolytes urinaires, etc...) après un traitement par la mélatonine (4, 5).

D'autres applications de la régulation des rythmes circadiens conférée par administration de la mélatonine sont notamment :
- le travail posté :
   il a été montré que la prise de mélatonine au moment du coucher des personnes soumises à différents types de travail posté (posté de 7 jours, changements progressifs, etc...) améliore la qualité et la durée du sommeil et la vigilance pendant la période d'activité (6) ;
- les sujets aveugles :
   ces sujets ne perçoivent plus la lumière, le principal synchroniseur de leur horloge circadienne. La plupart d'entre eux maintiennent tout de même un entraînement correct de cette horloge sur le rythme jour/nuit, probablement parce que d'autres signaux, telles les activités sociales, prennent le relais de l'environnement lumineux. Toutefois, l'horloge circadienne de certains aveugles n'est plus synchronisée et évolue en libre cours, c'est-à-dire avec sa période propre qui peut varier de 22 à 30 heures (7).
   Ceci conduit à une alternance de périodes favorables et défavorables pour ces sujets en fonction de la synchronisation ou désynchronisation par rapport aux cycles jour/nuit ; la mélatonine devrait donc permettre de synchroniser l'horloge circadienne de ces aveugles et faire disparaître cette alternance entre périodes favorables et défavorables ;
- insomnie par retard de phase :
   certains sujets souffrent du syndrome de "sommeil retardé". Ce syndrome se traduit par des difficultés pour s'endormir, un réveil difficile, une faible vigilance et un niveau de performance réduit. Ce syndrome résulte d'un décalage de l'horloge circadienne par rapport à ses synchroniseurs externes. La prise de mélatonine, environ 2 heures avant le coucher, réduit l'intervalle coucher-sommeil de 2 heures, ainsi que la qualité du sommeil (8) ;
   ceci s'applique également et de façon plus générale aux sujets qui ont un sommeil perturbé ou de mauvaise qualité en liaison ou non avec une pathologie, un syndrome de fatigue chronique, de l'anxiété, une dépression, ou avec l'âge du sujet, sachant que les concentrations nocturnes de mélatonine diminuent avec l'âge des sujets (9).
- le cancer :
   la mélatonine administrée de manière chronique durant l'après-midi (50 à 500 µg) peut inhiber la croissance des tumeurs cancéreuses de la mamelle de rates induites par le diméthylbenzanthracène (DMBA)(10).

Par ailleurs, des effets positifs de la mélatonine sur l'évolution de certains types de cancer (prostate, hypophyse, mélanome, leucémie, etc...) ont été observés expérimentalement chez le rat ou la souris (11) ; chez l'homme, des rapports ont suggéré l'efficacité thérapeutique de la mélatonine dans le traitement de carcinomes du poumon, de l'estomac ou du sein ou de leucémie (11, brevet US 4,855,305).

Il a également été montré qu'un effet de la mélatonine sur les stress saisonniers (12).

Les applications de la mélatonine et de ses dérivés sont très nombreuses et décrites dans différentes publications (brevets EP513702, EP438856, WO90/14084, WO88/07367, WO89/04659) et sont incorporées à cette demande de brevet à titre de référence.

Tous les dérivés de la mélatonine, analogues, homologues tels que décrits par exemple dans les brevets US 4,880,826, WO87/0432, WO89/04659 notamment, sont également incorporés par référence à la présente demande comme principes actifs avantageux de l'invention.

Il semble cependant qu'aucune voie d'administration décrite ne présente les qualités requises d'administration de ces composés pour notamment un rétablissement de l'horloge interne et des cycles circadiens, lesquelles qualités sont à la fois des qualités de confort puisque ce traitement s'adresse souvent à des personnes en bonne santé, et d'autre part, d'efficacité rapide, à court terme, et d'une durée suffisamment longue de telle façon qu'il y ait effectivement un rétablissement du rythme avec le rétablissement des différents marqueurs chronobiologiques tels que cités plus haut.

Différentes formes ont été proposées. On pourra citer à titre d'exemple la demande de brevet WO93/07870 qui est un dispositif pour administration transdermique de mélatonine. Cette demande de brevet revendique l'utilisation du système transdermique déjà connu et commercialisé, pour l'application particulière à la mélatonine.

La demande de brevet WO91/06290 est une composition bioadhésive homogène qui permet une adhésion sur les muqueuses, en particulier buccales, pendant un temps minimum de 6 heures et permettant une administration lente et contrôlée de la mélatonine par voie transmucosale exclusivement, il ne permet pas cependant une action rapide qui est nécessaire dans certaines circonstances.

La demande de brevet EP518468 est également relative à une formulation galénique à libération contrôlée de la mélatonine qui est un mode d'administration sélectionné parmi les formes orale, parentérale, rectale ou transdermique.

### - Description détaillée de l'invention :

Dans la description qui suit, on appellera indifféremment "composition galénique" ou "formulation galénique" toute association de constituants à but pharmacologique, prophylactique ou thérapeutique.

La mélatonine subit un effet de premier passage hépatique très important qui lui confère une biodisponibilité absolue (mesurée par le rapport des concentrations plasmatiques de mélatonine et par le rapport des aires sous les courbes respectives) faible ou variable. En effet, la comparaison des concentrations systémiques (ou salivaires) de mélatonine après administration orale comparées à celles obtenues après administration intraveineuse conduit à une biodisponibilité très variable d'un sujet à l'autre (13).

Comme les métabolites de la mélatonine et en particulier la N-acétyl sérotonine, le 5-méthoxy tryptamine et la 6-hydroxy mélatonine qui conduit par conjugaison à la 6-sulfatoxy mélatonine et à la 6-glucurono mélatonine (14) sont inactifs, il en résulte une grande variabilité des concentrations plasmatiques de la mélatonine et une biodisponibilité incomplète et/ou variable inter-individuellement.

Comme de plus les demi-vies biologiques de la mélatonine sont très courtes et voisines selon les auteurs de 10 minutes pour la première demi-vie et de 60 minutes pour la deuxième, cela conduit à rechercher un système d'administration évitant le premier passage hépatique (15).

Le système de l'invention est une composition ou formulation galénique à libération contrôlée mucoadhésive et caractérisée en ce qu'elle associe au moins deux couches,
- l'une (A) est mucoadhésive et permet une libération lente du principe actif et notamment de la mélatonine ou l'un de ses dérivés à la fois par voie transmucosale et par voie orale, notamment dans la salive si la composition galénique est implantée dans la bouche,
- l'autre (B) n'est pas mucoadhésive et permet une libération rapide du ou des principes actifs dans la lumière de la cavité dans laquelle est implantée la composition galénique et notamment dans la bouche.

Les vitesses de transfert lentes visées pour la première couche (A) sont de 0,02 mg/h à 1 mg/h selon les applications ; les vitesses de transfert rapides visées pour la couche (B) sont de 0,2 mg/h à 10 mg/h selon les applications.

Par le terme de "rapide" pour la couche à libération rapide on entend une libération flash, immédiate qui peut se prolonger pendant 2 à 5 heures. Cette couche non mucoadhésive permet donc de libérer une certaine quantité de mélatonine nécessaire à donner le top chronobiologique clair.

Autrement dit, la composition galénique de l'invention bicouche mucoadhésive présente à la fois les avantages d'un confort d'utilisation, de pouvoir conférer le top chronobiologique clair du démarrage souhaité de l'action ; ce top est donné par une dose de charge dont la rapidité est suffisante pour conduire à des concentrations plasmatiques élevées de mélatonine de façon qu'elles soient largement supérieures au seuil de réponse de l'ensemble des sujets et deviennent de ce fait naturellement reconnues comme telles ; le relais est assuré par la couche dont un côté est mucoadhésif qui, lui, confère une libération contrôlée par les voies mucosales ou sublinguales et buccales citées plus haut ; le système en outre permet une adhésion à la membrane, supérieure à 10 heures, et le système peut être retiré à volonté au temps final désiré conduisant à un arrêt immédiat des effets de la mélatonine.

La composition galénique de l'invention est particulièrement avantageuse lorsqu'elle est placée dans la cavité buccale, mais il va de soi qu'elle peut-être adaptable à toute cavité couverte de muqueuse telles les cavités anale ou vaginale.

Il apparaît clairement que ce système bicouche est particulièrement avantageux pour tous les composés actifs nécessitant une transmission rapide par flash dans le système circulatoire suivie d'une administration lente et contrôlée pendant plusieurs heures.

La bicouche est un des exemples d'une formulation galénique hétérofonctionnelle. D'autres types de formulations galéniques hétérofonctionnelles peuvent être mises en oeuvre, dont les fonctions de libération rapide, semi-rapide, lente ou très lente qui peuvent être associées dans la même formulation : à titre d'exemple, des formulations telles que décrites dans (16) dans lesquelles des microgranules fluidisées, de formulations différentes pourraient être mélangées et compressées avec un copolymère d'acide polyacrylique et d'alkylsucrose, ou Cabopol®, ou produit équivalent ; le résultat en serait également une formulation galénique mucoadhésive hétérofonctionnelle associant une libération rapide et une libération lente d'un même principe actif.

La composition galénique selon l'invention est plus particulièrement caractérisée en ce que la première couche (A) mucoadhésive contient au moins :
- une résine polymérique biocompatible, notamment du carbopol 934P® et/ou polyvinyl-pyrrolidone (POVIDONE) ou tout autre polymère adhésif biocompatible, ces deux résines agissant à titre d'agents d'adhésion et entrent dans cette couche à raison de 35 à 80% en poids de ladite couche et de préférence 50 à 75% en poids,
- un agent liant ou diluant à raison de 5 à 40% en poids de la bicouche mucoadhésive et de préférence de 10 à 30% ; un tel exemple d'agent liant est le phosphate dicalcique (ENCOMPRESS) ou d'autres liants de type hydroxypropylcellulose, hydroxypropylméthylcellulose ou tout autre type d'ingrédient décrit dans la Pharmacopée US XXII ou USP XXII, p. 1857-1859,
- du méthocel BASF ou tout autre agent retard de type hydroxypropylméthylcellulosique ou équivalent, ne nécessitant pas d'adjonction d'eau à une concentration en poids de 3 à 20% et de préférence de 5 à 15%,
- du stéarate de magnésium à titre de lubrifiant à une concentration maximum de 1%, tout type de lubrifiant tel que cité dans la Pharmacopée US XXII, p. 1858, convenant également comme composant de cette couche mucoadhésive,
- de l'Aérosil 200 à titre d'agent d'écoulement ou tout équivalent telle la silice colloïdale anhydre (Carboxyl, siloïde), lequel est ajouté à la couche à une concentration maximum de 1% et de préférence à celle de 0,2%,
- la mélatonine ou l'un de ses dérivés tels que cités plus haut est ajoutée à la concentration de 0,05 à 2%, et de préférence de 0,3 à 1,5%.

Il est préférable de maintenir le niveau d'humidité faible dans la couche mucoadhésive de la composition galénique de l'invention.

Une telle couche bioadhésive a un contenu en eau de moins de 10% en poids, et de préférence moins de 6% et de préférence encore moins de 3% du poids total de la couche.

Comme cela sera indiqué dans l'exemple de réalisation indiqué plus loin, il est donc préférable de réaliser l'ensemble de cette forme galénique en atmosphère sèche ou en humidité relative inférieure à 30%.

La couche à libération rapide (B) contient au moins :
- une résine polymérique de type Carbopol® tel que décrite ci-dessus comme agent de liaison ou tout autre agent assurant la même fonction à la concentration en poids de 2 à 15%, et de préférence de 8 à 12% ;
- un diluant de type lactose qualité Fast Flow ou tout type de diluant tel que décrit dans la table de référence ;
- un désintégrant de type Ac Di Sol qui est une carboxyméthylcellulose réticulée à la concentration de 1 à 30%, et de préférence de 3 à 15% : il faut noter que, pour ce produit, plus le pourcentage est élevé, plus le relargage est rapide ; un pourcentage de 30% confère un relargage très rapide du principe actif, alors qu'un pourcentage de 1% donne un relargage très lent de l'ordre de 5 heures du principe actif ;
- la mélatonine est également ajoutée dans cette couche à libération rapide à la concentration de 0,2 à 10%, et de préférence de 1 à 6%.

En option, il est possible d'ajouter un colorant pour distinguer cette couche de l'autre, lequel colorant doit être ajouté à un maximum de 1% en poids du poids des composés de cette couche.

Les tableaux 1 et 2 ci-dessous montrent une composition type de chacune des couches (A) et (B) de l'invention contenant la mélatonine à titre de principe actif.

**TABLEAU 1**

| COUCHE MUCOADHESIVE A LIBERATION PROLONGEE | | | | |
|---|---|---|---|---|
| | Pourcentage | Formule unitaire | Role des constituants | Pourcentage limite |
| Méthocel | 10% | 15 mg | agent retard | 3-20 |
| Mélatonine | 1% | 1,5 mg | principe actif | variable |
| Emcompress | 22,5% | 33,7 mg | liant-diluant | 5-40 |
| Carbopol 934 P® | 65,3% | 98 mg | agent d'adhésion | 35-80 |
| Stéarate de Mg | 1% | 1,5 mg | lubrifiant | max 1% |
| Aérosil 200 | 0,2% | 0,3 mg | agent d'écoulement | max 1% |
| | 100% | 150 mg | | |

**TABLEAU 2**

| COUCHE A LIBERATION RAPIDE | | | | |
|---|---|---|---|---|
| | Pourcentage | Formule unitaire | Role des constituants | Pourcentages limites |
| Ac Di Sol | 11% | 5,5 mg | désintégrant | 1-30 |
| Lactose fast flow | 74% | 37 mg | diluant | variable |
| Carbopol 934P® | 11% | 5,5 mg | agent de liaison | 2-15 |
| Mélatonine | 3% | 1,5 mg | principe actif | variable |
| Colorant | 1% | 0,5 mg | | max 1% |
| | 100% | 50 mg | | |

L'invention est relative enfin à l'utilisation d'une telle composition galénique comme médicament pour le traitement des déréglements du sommeil, pour le traitement de l'anxiété et notamment de l'anxiété saisonnière, pour le traitement du cancer et contre le vieillissement.

Les exemples ci-après sans caractère limitatif montreront tout l'intérêt de ce type de composition mucoadhésive bicouche contenant de la mélatonine pour un rétablissement rapide et durable du taux de mélatonine dans le plasma. Cet exemple n'est bien évidemment pas limitatif de l'utilisation de la mélatonine, ni de la composition particulière de la composition bicouche utilisée.

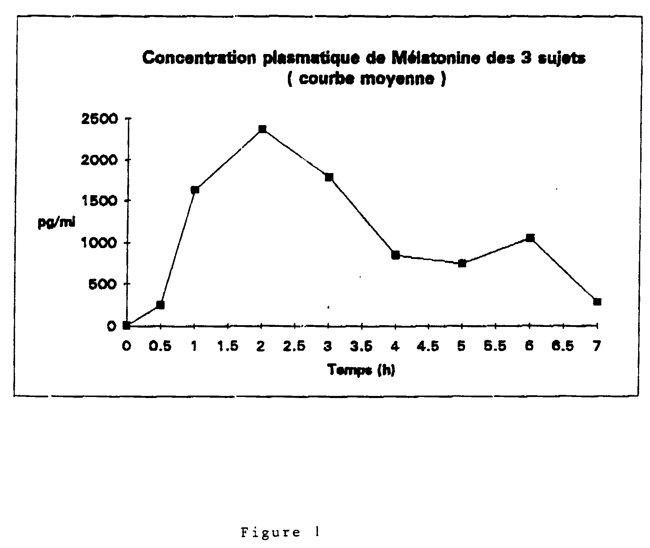

Dans cet exemple, la figure 1 représente le dosage plasmatique de trois sujets ayant fixé la composition bicouche à l'intérieur de la cavité buccale, sur la face interne de la lèvre inférieure, au temps 0 et l'ayant retiré au temps 7 heures. En abscisse est représenté le temps, et en ordonnée le concentration de mélatonine en pg/ml (picogrammes par millilitre).

### - EXEMPLE I : fabrication de la composition mucoadhésive bicouche :

Le procédé de fabrication de la composition mucoadhésive bicouche se fait, dans un premier temps, par la fabrication de la couche à libération immédiate ou semi-lente non mucoadhésive, puis de la couche mucoadhésive suivie enfin par une compression finale des deux couches. Il va de soi que la variante dans laquelle on fabrique d'abord la couche mucoadhésive, puis la couche à libération immédiate, suivie par la compression finale des deux couches, est équivalente.

La composition réalisée dans l'exemple qui suit est celle représentée dans les tableaux 1 et 2 du point de vue des pourcentages de chacun des constituants.
- Fabrication de la couche à libération rapide ou semi-lente :
   On introduit dans un mélangeur Turbula ou équivalent le lactose fast flow, le Carbopol® (préalablement tamisé), la mélatonine et le colorant; le tout est mélangé 10 minutes.
   Il est ajouté ensuite de l'Ac Di Sol ou tout autre agent désintégrant équivalent et le tout est mélangé 10 minutes.
   Le mélange est ensuite stocké dans un récipient adapté et pesé.
- Fabrication de la couche mucoadhésive :
   Cette couche est faite par granulation par voie sèche et comprend les étapes suivantes :
   1. Le Carbopol 934P® est introduit dans un mélangeur Turbula avec l'alcool (Aérosil) de sang, le tout étant mélangé 5 minutes.
   2. Le mélange est ensuite tamisé sur un diamètre de grille de 500 µm.
   3. Le mélange ainsi tamisé est introduit dans un mélangeur de type Turbula et mélangé pendant 3 minutes.
   4. La mélatonine est ensuite ajoutée, ainsi que le Méthocel, au mélange ci-dessus et agitée 10 minutes.
   5. Puis, la moitié du Stéarate de magnésium ou autre lubrifiant équivalent préalablement tamisé est ajoutée et le tout mélangé pendant 3 minutes.
   6. Puis, le mélange est récupéré, pesé et stocké dans un récipient adapté.
- Compaction ou première compression :
   7. Le mélange de l'étape 6 est introduit dans le trémie d'alimentation d'un compacteur.
   8. Les "galets" de l'étape 7 sont granulés sur un granulateur de type Erweka ou équivalent, équipé d'une grille de diamètre de 1 mm.
   9. Le grain de l'étape 8 est introduit dans un mélangeur type Turbula ; l'encompress est ajouté et le tout mélangé pendant 10 minutes.
   10. Au mélange de l'étape 9 est ajoutée la deuxième moitié du lubrifiant, par exemple le stéarate de magnésium, préalablement tamisé et le tout est mélangé pendant 3 minutes.
   11. Ce mélange 10 est stocké dans un récipient adapté et pesé.
- Réalisation de la couche mucoadhésive : deuxième compression :
   12. Le grain de l'étape 11 est reconditionné et introduit dans la trémie d'alimentation de la machine à compacter et la compression est ainsi initiée. Pour cette étape, les comprimés devront avoir une dureté suffisante pour avoir une friabilité inférieure à 1%, mais une aptitude à la compression doit exister pour permettre la réalisation du comprimé final.
- Compression finale :
   1. Les comprimés mucoadhésifs sont introduits dans la première trémie d'alimentation et le mélange de type libération immédiate est introduit dans la deuxième trémie.
   2. La quantité de poudre qui doit alimenter la chambre de compression est réglée.
   3. La compression finale est réalisée avec un suivi de la dureté et du poids de la composition finale.
      Toute cette fabrication doit être réalisée en bloc sec, c'est-à-dire HR inférieur à 30%.

### - EXEMPLE II : effet de l'administration de la composition galénique de l'invention sur le taux plasmatique de mélatonine de sujets normaux :

Les concentrations plasmatiques de mélatonine ont été étudiées chez 3 individus traités avec le comprimé présenté en exemple I.

Trois sujets; 2 hommes et une femme âgés respectivement de 31, 50 et 50 ans ont présenté des concentrations de mélatonine au jour J-1 à T0 (9 h 30), T0 + 1 h (10 h 30) et T0 + 4 h (13 h 30) inférieurs à la limite de quantification de la méthode RIA décrite ci-après. Le jour suivant, à J0, ils ont appliqué à T0 (9 h 30) le comprimé mucoadhésif à l'intérieur de la lèvre inférieure à la hauteur de l'incisive et l'ont gardé jusqu'au temps T0 + 6 h (16 h 30).

Le comprimé était dosé à 1,5 mg de mélatonine dans la couche mucoadhésive à libération programmée et à 1,5 mg de mélatonine dans la couche extérieure ou dose charge.

Des prélèvements sanguins en tubes héparinés ont été réalisés aux temps T0 (avant mise en place du comprimé) et à T0 + 0.5 h, T0 + 1 h, T0 + 2 h, T0 + 3 h, T0 + 4 h, T0 + 5 h, T0 + 6 h, T0 + 7 h. Après centrifugation et recueil du plasma, un dosage de la mélatonine a été réalisé par méthode RIA (17, 18) dont les anticorps sont en provenance de Stock Grand (UK) avec traceur tritié (Amersham). Cette méthode permet une détermination des concentrations entre 10 pg/ml et 200 pg/ml. La limite de quantification était de 10 pg/ml. Des dilutions adéquates ont été réalisées pour le dosage des concentrations plasmatiques de la mélatonine sur les trois profils.

Les concentrations mesurées ont été les suivantes (cf. tableau ci-dessous) et la courbe de la figure 1 représente la concentration moyenne des sujets.

| Temps (h) | Concentration moyenne (pg/ml) |
|---|---|
| 0 | 0 |
| 0.5 | 254 |
| 1 | 1632 |
| 2 | 2375 |
| 3 | 1790 |
| 4 | 850 |
| 5 | 750 |
| 6 | 1050 |
| 7 | 283 |

Il a été démontré que les concentrations diurnes de mélatonine sont faibles, ce qui a été vérifié sur les 3 sujets pour lesquels les concentrations étaient inférieures a la limite de quantification (< 10 pg/ml) à J-1. L'administration ayant été réalisée à J0 de 9 h 30 à 16 h 30, les concentrations plasmatiques rencontrées sur ces 3 sujets sont le reflet de la mélatonine exogène administrée par le comprimé mucoadhésif.

Le comprimé a été parfaitement toléré; aucune gêne n'étant ressentie un quart d'heure après la pose et jusqu'à son enlèvement. Les sujets ont ressenti l'envie de dormir 2 h après la pose du comprimé caractérisée chez les 3 sujets par l'envie de faire la sieste pour l'un, un baillement inhabituel pour l'autre et la sensation d'avoir dormi 3 heures la veille (malgré une bonne nuit) pour le dernier. Aucune gêne n'a été ressentie pour la prise du repas de 13 h à 13 h 30 (boisson et nourriture). Le comprimé a été retiré avec action mécanique du doigt. Il n'a résulté de cette administration aucun effet secondaire général ou local montrant une excellente tolérance clinique du comprimé.

### - CONCLUSION :

De telles compositions galéniques telles que décrites ci-dessus dans l'invention sont particulièrement avantageuses à plusieurs titres.

Le premier est le confort d'utilisation puisque les composés qui adhèrent à l'intérieur de la bouche au niveau de la muqueuse sont relativement stables, permettent une alimentation normale et ne sont pas gênants pour les sujets.

Un autre avantage est que, sitôt le comprimé enlevé, les concentrations plasmatiques de mélatonine décroissent pour devenir inférieures à la limite de quantification.

L'avantage essentiel apparaît clairement à l'observation de la figure 1 qui indique bien l'effet immédiat de flash conféré grâce à la couche (B) non mucoadhésive suivi d'un relargage contrôlé et continu grâce à la deuxième couche mucoadhésive. On pourra, à titre de comparaison, comparer cette courbe aux courbes données par exemple dans le brevet EP518468 dans lequel l'apparition de la mélatonine dans le plasma est relativement lente, mimant le cycle normal de l'apparition et de la disparition au cours de la nuit.

Les cinétiques plasmatiques de mélatonine données dans les figures 5 et 6 de la demande de brevet WO93/07870 font apparaître une lente augmentation de la concentration de la mélatonine dans le plasma (voir notamment la figure 6 et la figure 8 dudit brevet).

La même observation peut être faite quant aux cinétiques plasmatiques de la mélatonine obtenues à partir de la composition bioadhésive décrite dans la demande de brevet WO91/06290 qui montrent une augmentation lente et continue de la mélatonine dans le plasma, du fait de la libération prolongée du principe actif.

Aucun système n'a montré le double avantage du passage immédiat suivi d'une libération semi-lente et contrôlée pendant toute la durée du maintien de la composition galénique de l'invention sur la muqueuse.

### BIBLIOGRAPHIE

1. Lewy A.J., Wehr T.A., Goodwin F.K., Newsome D.A., Markey S.P., 1980. Light suppresses melatonin secretion in humans. Science 210:1267.
2. Arendt J., Broadway J., 1987. Light and melatonin as zeitheibers in man.
3. Arendt J., Aldhous M., English J., Marks V., Arendt K.H., Marks M; and Folkard S., 1987. Some effects of jet lag and their alleviation by melatonin. Ergonomics, 30:1379.
4. Samel A., Maas H., Vejroda M., Wegman H.M., 1989. Influence of melatonin treatment on human circadian rhythmicity. in "Aviation, Space and Environmental Medicine" Abst 485, p. 52.
5. Nickelsen T., Land A., Bergau L., 1991. The effect of 6-,9- and 11-hour time shifts on circadian rhythms: adaptation of sleep parameters and hormonal patterns following the intake of melatonin or placebo. In 'Advances in Pineal Research: 5", Arendt J., Pévert P. (Eds), John Libbey & Co Ltd, p. 306.
6. Folkard S. Arendt J., Clark M., 1990. Can melatonin improve shift workers' tolerance of the night shift ? Some preliminary findings. Interdisciplinary Cycle Research, Proceeding of the European Society for Chronobiology.
7. Aldhous M., Arendt J., 1991. Assessment of melatonin rhythms and the sleep-wake cycle in blind subjects. in 'Advances in Pineal Research:5', Arendt J., Pévet P. (Eds), John Libbey & Co Ltd, p. 307.
8. Tzischinsky O., Dagan Y., Lavie P., 1993. The effects of melatonin on the timing of sleep in patients with delayed sleep phase syndrome. in "Melatonin and the pineal gland-From basic science Publishers P.V., p. 351.
9. Touitou Y. et al., 1981. "Age- and Mental Health-Related Circadian Rhythms of Plasma Levels of Melatonin, Prolactin, Luteinizing Hormone and Follicle-Stimulating Hormone in Man", J. Endocr. 91:467-475.
10. Tamarkin L., Almeida O.F.X., Danforth D.N., 1985. Melatonin and malignant disease.
11. Blask D.E., 1993. Melatonin in oncology. In "Melatonin: biosynthesis, physiological effects, and clinical applications", Yu H.S., Reiter R.J. (Eds), CRC Press, Boca Raton, Floride, p. 447.
12. Wirz. Justice et al. (1990). J. Psychiat. Res. 24(2):129-137.
13. Waldhauser F. Waldhauser M., Lieberman R. et al., 1984. "Bioavailability of Oral Melatonin in Human". Neuroendocrinology 39:307.313.
14. Hing Sing Yu 1993.
15. Mallo C., Zaïdan R., Galyg et al. 1990. "Pharmacokinetics of Melatonin in Man after Intravenous Infusion and Bolus Injection". Eur. J. Clin. Pharmacol. 38:297-301.
16. RITSCHEL W.A. et al. Peroral solid dosage forms with prolonged action in DRUG DESIGN, vol. 4 (E.J. ARIEN Ed.), Academic New York, 1973, chap. 2, p. 37.
17. Arendt J., Sizonenko P.C., Pauniner L. "Melatonin Radioimmunoassay". 1975. J. Clin. Endocrinol. Metab. 40:347-350.
18. Howanitz P.J., Howanitz J.H. 1983. "Direct Radioimmunoassay for Melatonin in Plasma". Clin. Chem. No. 2, 29:396-397.

## Revendications

1. Formulation galénique mucoadhésive hétérofonctionnelle :
- contenant un principe actif choisi dans le groupe constitué par la mélatonine, ou les dérivés de la mélatonine,
- associant au moins deux couches :
- l'une (A) est mucoadhésive et permet une libération prolongée du ou des principes actifs à la fois par voie transmucosale et par voie orale,
- l'autre (B) non mucoadhésive permet une libération immédiate ou semilente par voie orale du ou des principes actifs,
caractérisée en ce que
a) la couche (A) mucoadhésive contient au moins :
- un agent d'adhésion choisi dans le groupe constitué d'un co-polymère biocompatible d'acide polyacrylique et d'alkylsucrose et/ou de polyvinyl pyrrolidone à la concentration en poids de 35 à 80%,
- un liant diluant de type hydroxyméthylcellulose ou hydroxypropylcellulose, ou le phosphate dicalcique à une concentration en poids de 5 à 40%,
- un agent retard de type hydroxypropylméthylcellulose à une concentration en poids de 3 à 20%,
- un lubrifiant de type stéarate de magnésium à un maximum de 1% en poids,
- un agent d'écoulement de type silice colloïdale anhydre (carboxyl, siloïde) ou dioxyde de silice colloïdal à une concentration maximale de 1%,
- un principe actif à une concentation compatible avec l'effet pharmacologique recherché,
b) la couche (B) contient au moins :
- un agent de liaison de type copolymère d'acide polyacrylique et d'alkylsucrose ou de polyvinylpyrrolidone à la concentration de 2 à 15%,
- un désintégrant de type carboxyméthylcellulose réticulée à la concentration de 1 à 30%,
- le principe actif à une concentration efficace pour l'effet pharmacologique recherché,
- un diluant type lactose fast flow de 60 à 80%,
- le cas échéant, un colorant à la concentration maximum de 1%.

2. Formulation selon la revendication 1, caractérisée en ce que le principe actif est la mélatonine ou l'un quelconque de ses dérivés, actifs à la dose de 0,05 à 2%.

3. Formulation selon la revendication 1 ou 2, caractérisée en ce que le principe actif est la mélatonine ou l'une quelconque de ses dérivés, actifs à la dose de 0,3 à 1,5%.

4. Formulation selon une des revendications 1 à 3, caractérisée en ce que l'agent retard est présent à la concentration de 8 à 12%.

5. Formulation selon une des revendications 1 à 4, caractérisée en ce que le liant diluant est présent à la concentration de 15 à 30%.

6. Formulation selon une des revendications 1 à 5, caractérisée en ce que l'agent d'adhésion est le carbopol ® à la concentration de 45 à 75% dans la couche (A) et de 8 à 12% dans la couche (B).

7. Utilisation d'une formulation galénique mucoadhésive hétérofonctionnelle selon l'une des revendications 1 à 6 dans la fabrication d'un médicament permettant la restauration rapide et prolongée dans le plasma des mammifères du principe actif contenu dans ladite formulation.

8. Utilisation selon la revendication 7, caractérisée en ce que le principe actif est la mélatonine ou l'un de ses dérivés, dont la dose se situe entre 0.05 et 2%.

9. Utilisation selon la revendication 8, caractérisée en ce que le principe actif est la mélatonine ou l'un de ses dérivés, dont la dose se situe entre 0.3 et 1,5%.

10. Utilisation selon l'une des revendications 7 à 9, dans la fabrication d'un médicament pour le traitement des déréglements du sommeil.

11. Utilisation selon l'une des revendications 7 à 9, dans la fabrication d'un médicament pour le traitement de l'anxiété, et notamment de l'anxiété saisonnière.

12. Utilisation selon l'une des revendications 7 à 9, dans la fabrication d'un médicament pour le traitement du cancer.

13. Utilisation selon l'une des revendications 7 à 9, dans la fabrication d'un médicament contre le vieillissement.

## Claims

1. A heterofunctional mucoadhesive galenical formulation:
• containing an active principle selected from the group formed by melatonin and melatonin derivatives;
• combining at least two layers;
• one (A) being mucoadhesive and permitting sustained release of the active principle or principles both transmucosally and orally;
• the other (B), non-mucoadhesive, permitting an immediate or semi-slow oral release of the active principle or principles;
characterized in that
a) mucoadhesive layer (A) contains at least:
• an adhesion agent selected from the group formed by a biocompatible copolymer of polyacrylic acid and alkylsucrose, and/or polyvinylpyrrolidone, in a concentration of 35% to 80% by weight;
• a hydroxymethylcellulose or hydroxypropylcellulose type diluent binding agent, or dibasic calcium phosphate, in a concentration of 5% to 40% by weight;
• a hydroxypropylmethylcellulose type retarding agent in a concentration of 3% to 20% by weight;
• a magnesium stearate type lubricant in a maximum concentration of 1% by weight;
• an anhydrous colloidal silica (Carboxyl, Siloid) or colloidal silicon dioxide type glidant in a maximum concentration of 1%;
• an active principle in a concentration which is compatible with the desired pharmacological effect;
b) layer (B) contains at least:
• a copolymer of polyacrylic acid and alkylsucrose or polyvinylpyrrolidone type binding agent in a concentration of 2% to 15%;
• a cross-linked carboxymethylcellulose type disintegrant in a concentration of 1% to 30%;
• the active principle in a concentration which is effective for the desired pharmacological effect;
• a Fast Flow lactose type diluent, in a concentration of 60% to 80%;
• where appropriate, a colorant in a maximum concentration of 1%.

2. A formulation according to claim 1, characterized in that the active principle is melatonin or any one of its derivatives which are active in a dose of 0.05% to 2%.

3. A formulation according to claim 1 or claim 2, characterized in that the active principle is melatonin or any one of its derivatives which are active in a dose of 0.3% to 1.5%.

4. A formulation according to any one of claims 1 to 3, characterized in that the retarding agent is present in a concentration of 8% to 12%.

5. A formulation according to any one of claims 1 to 4, characterized in that the diluent binding agent is present in a concentration of 15% to 30%.

6. A formulation according to any one of claims 1 to 5, characterized in that the adhesion agent is Carbopol® in a concentration of 45% to 75% in layer (A) and 8% to 12% in layer (B).

7. Use of a heterofunctional mucoadhesive galenical formulation according to any one of claims 1 to 6, for the production of a drug to rapidly restore and sustain the active principle contained in said formulation in mammalian plasma.

8. Use according to claim 7, characterized in that the active principle is melatonin or one of its derivatives, the dose of which is in the range 0.05% to 2%.

9. Use according to claim 8, characterized in that the active principle is melatonin or one of its derivatives, the dose of which is in the range 0.3% to 1.5%.

10. Use according to my one of claims 7 to 9, for the production of a drug for the treatment of sleep disorders.

11. Use according to any one of claims 7 to 9, for the production of a drug for the treatment of anxiety, and in particular of seasonal anxiety.

12. Use according to any one of claims 7 to 9, for the production of a drug for the treatment of cancer.

13. Use according to any one of claims 7 to 9, for the production of a drug for counteracting ageing.

## Patentansprüche

1. Mukoadhäsive heterofunktionelle galenische Formulierung:
- enthaltend einen Wirkstoff, ausgewählt aus der Gruppe, gebildet aus Melatonin oder den Derivate von Melatonin,
- in Verbindung mit mindestens zwei Schichten:
- die eine (A) ist mukoadhäsiv und erlaubt eine Freisetzung über längere Zeit des oder der Wirkstoffe zugleich auf transmukosalem Weg und auf oralem Weg,
- die andere, nicht mukoadhäsive (B) erlaubt eine sofortige oder halbschnelle Freisetzung des oder der Wirkstoffe auf oralem Weg,
dadurch gekennzeichnet, daß
(a) die mukoadhäsive Schicht (A) mindestens enthält:
- ein Haftmittel, ausgewählt aus der Gruppe, gebildet aus einem biokompatiblen Copolymer von Polyacrylsäure und Alkylsucrose und/oder Polyvinylpyrrolidon in einer Konzentration von 35 bis 80 Gew.-%,
- ein verdünnendes Bindemittel vom Typ Hydroxymethylcellulose oder Hydroxypropylcellulose oder Dicalciumphosphat in einer Konzentration von 5 bis 40 Gew.-%,
- ein Verzögerungsmittel vom Typ Hydroxypropylmethylcellulose in eine Konzentration von 3 bis 20 Gew.-%,
- ein Gleitmittel vom Typ Magnesiumstearat zu maximal 1 Gew.-%,
- ein Fließmittel vom Typ wasserfreies kolloidales Siliciumdioxid (Carboxyl, siloid/soloid) oder kolloidales Siliciumdioxid in einer maximalen Konzentration von 1%,
- einen Wirkstoff in einer mit der angestrebten pharmakologischen Wirkung verträglichen Konzentration,
(b) die Schicht (B) mindestens enthält:
- ein Verbindungsmittel vom Typ Copolymer von Polyacrylsäure und Alkylsucrose oder Polyvinylpyrrolidon in einer Konzentration von 2 bis 15%,
- ein Aufschlußmittel vom Typ vernetzte Carboxymethylcellulose in einer Konzentration von 1 bis 30%,
- den Wirkstoff in einer für die angestrebte pharmakologische Wirkung wirksamen Konzentration,
- ein Verdünnungsmittel vom Typ fast-flow-Lactose zu 60 bis 80%,
- gegebenenfalls ein Färbemittel in einer maximalen Konzentration von 1%.

2. Formulierung nach Anspruch 1, dadurch gekennzeichnet, daß der Wirkstoff Melatonin oder irgendeines seiner Derivate, die in einer Dosis von 0,05 bis 2% aktiv sind, ist.

3. Formulierung nach Anspruch 1 oder 2, dadurch gekennzeichnet, daß der Wirkstoff Melatonin oder irgendeines seiner Derivate, die in einer Dosis von 0,3 bis 1,5% aktiv sind, ist.

4. Formulierung nach einem der Ansprüche 1 bis 3, dadurch gekennzeichnet, daß das Verzögerungsmittel in einer Konzentration von 8 bis 12% vorliegt.

5. Formulierung nach einem der Ansprüche 1 bis 4, dadurch gekennzeichnet, daß das verdünnende Bindemittel in einer Konzentration von 15 bis 30% vorliegt.

6. Formulierung nach einem der Ansprüche 1 bis 5, dadurch gekennzeichnet, daß das Haftmittel Carbopol® in einer Konzentration von 45 bis 75% in der Schicht (A) und von 8 bis 12% in der Schicht (B) ist.

7. Verwendung einer mukoadhäsiven heterofunktionellen galenischen Formulierung nach einem der Ansprüche 1 bis 6 bei der Herstellung eines Arzneimittels, das die schnelle und über längere Zeit andauernde Wiederherstellung des in der genannten Formulierung enthaltenen Wirkstoffs in dem Plasma von Säugetieren ermöglicht.

8. Verwendung nach Anspruch 7, dadurch gekennzeichnet, daß der Wirkstoff Melatonin oder eines seiner Derivate, deren Dosis zwischen 0,05 und 2% liegt, ist.

9. Verwendung nach Anspruch 8, dadurch gekennzeichnet, daß der Wirkstoff Melatonin oder eines seiner Derivate, deren Dosis zwischen 0,3 und 1,5% liegt, ist.

10. Verwendung nach einem der Ansprüche 7 bis 9 bei der Herstellung eines Arzneimittels für die Behandlung von Schlafstörungen.

11. Verwendung nach einem der Ansprüche 7 bis 9 bei der Herstellung eines Arzneimittels für die Behandlung von Angst und insbesondere saisonaler Angst.

12. Verwendung nach einem der Ansprüche 7 bis 9 bei der Herstellung eines Arzneimittels für die Behandlung von Krebs.

13. Verwendung nach einem der Ansprüche 7 bis 9 bei der Herstellung eines Arzneimittels gegen das Altern.
